(19) Europäisches Patentamt
European Patent Office
Office européen des brevets

(11) Publication number: **0 051 209**
**B1**

# (12) EUROPEAN PATENT SPECIFICATION

(45) Date of publication of patent specification: **16.01.85**

(51) Int. Cl.$^4$: **G 01 N 33/96,** G 01 N 33/64

(21) Application number: **81108607.3**

(22) Date of filing: **21.10.81**

## (54) Device and method for preparation of a control solution for ketone determination.

(30) Priority: **03.11.80 US 202924**

(43) Date of publication of application:
**12.05.82 Bulletin 82/19**

(45) Publication of the grant of the patent:
**16.01.85 Bulletin 85/03**

(84) Designated Contracting States:
**DE GB**

(56) References cited:
**EP-A-0 029 915**
**US-A-4 172 049**
**US-A-4 193 766**

(73) Proprietor: **MILES LABORATORIES, INC.**
**1127 Myrtle Street**
**Elkhart Indiana 46514 (US)**

(72) Inventor: **Sheats, John Eugene**
**173 Carter Road**
**Princeton New Jersey 08540 (US)**
Inventor: **Dombrowski, Kenneth Edward**
**127 Elton Avenue**
**Yardville New Jersey 08620 (US)**

(74) Representative: **Senftl, Hannes, Dr. et al**
**c/o Bayer AG Konzernverwaltung RP**
**Patentabteilung**
**D-5090 Leverkusen Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 051 209 B1

**Description**

This invention relates to a device for use in the preparation of a ketone control solution and to a method for preparing it.

Background of the invention

When a procedure is devised for determining the presence of a sample constituent—be the devised procedure gravimetric, volumetric, spectrophotometric or whatever mode—its efficacy in producing reliable results must somehow be assessed. Otherwise, the data developed is meaningless. Hence, devising such a procedure extends far beyond building a machine, formulating reagents or developing a technique. It also must of necessity include evaluating experimental error. There must be a way of predicting the dependability of the data produced by the procedure.

The easiest, most direct way to study parameters such as reproducibility, sensitivity, accuracy and need for calibration is to subject the procedure to a test sample wherein the analyte presence and/or concentration is known beforehand, i.e., a control solution. The data furnished by the procedure can then be compared with known data and any discrepancies properly noted.

The present invention concerns itself with assessing procedures for determining the presence and/or concentration of ketone bodies in a liquid sample. Moreover, it relates to a device for preparing a ketone control solution for use in assessing the performance of various ketone body determination procedures.

Description of the prior art

Acetoacetic acid (acetylacetic acid) is a normal end product of fatty acid oxidation in the liver. It is also produced to a very limited extent by oxidative breakdown of leucine, phenylalanine, and tyrosine. $\beta$-hydroxybutyric acid is formed from acetoacetic acid by reversible reduction. Acetone is produced through nonreversible decarboxylation of acetoacetic acid.

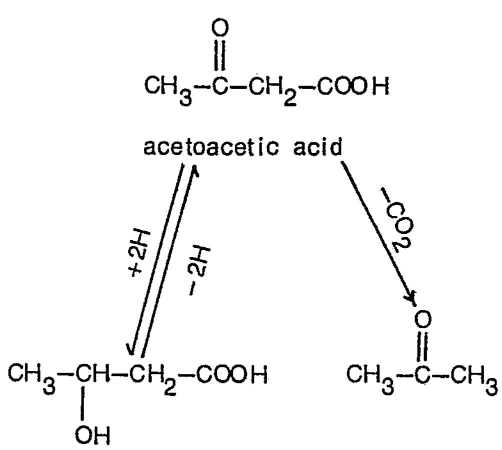


acetoacetic acid

$\beta$-hydroxybutyric acid acetone

These three substances are commonly referred to as ketone bodies or acetone bodies.

Abnormally high amounts of ketone bodies in urine or blood are referred to as *ketonuria* and *ketonemia*, respectively. These conditions can occur as a result of such diverse pathological conditions as diabetes mellitus and starvation. Because of this disease/symptom relationship, especially with diabetes mellitus, there is keen interest in the determination of ketone bodies in urine. In the case of a diabetic, the detection of ketonuria is of great significance, since a change in insulin dosage or other disease management is often indicated. Thus, strong emphasis has been placed by the medical profession on ketone body analysis, resulting in the development of a plurality of procedures sensitive to the presence and/or concentration of ketone bodies in urine.

But interest in monitoring the presence of ketone is by no means restricted to the medical profession. These compounds find a myriad of industrial applications—they are used as solvents in nitrocellulose coatings and vinyl films, they find applications in paint removers, cleaning fluids, organic synthesis, explosives manufacture, and as food additives—and in each there is a need at one time or another to perform an analytical procedure to determine ketone presence and/or concentration. These and numerous other concerns with respect to ketone presence have engendered many ketone tests.

One such test takes advantage of the propensity of ketones to react with sodium nitroprusside to give intense colors. Thus, acetone when treated with nitroprusside produces an intense red-yellow color which changes to pink-violet on acidification with acetic acid [see Fritz Feigl, *Spot Tests in Organic Analysis*, 7th ed. (1966)]. This phenomenon occurs as a result of a coupling reaction through the NO group of the nitroprusside and the ketone to yield an isonitrosoketone which remains in the reaction mixture as a complex, colored anion. The iron (III) of the nitroprusside is reduced to its divalent state (II). It has been found that ketones which do not contain methyl or methylene groups bound to carbonyl groups are not reactive, or at least they do not produce colorforms, with nitroprusside.

The same or similar chemistry can be found in the ketone-sensitive portion of analytical reagent strips known as N-Multistix® and Keto-Diastix® and in the reagent tablet, Acetest®, all of which are marketed by the Ames Division of Miles Laboratories, Inc. All three of these devices for determining ketone bodies are based on the nitroprusside-ketone complexing phenomenon. Thus, when the reagent strips are immersed in an aqueous ketone solution, or when the tablet is contacted with such a solution, the formation of a colored complex indicates the presence of a ketone. Moreover, the concentration of ketone can be estimated

based on the intensity and hue of the color formed.

As stated *supra*, these and other methods for ketone body estimation require a way of estimating their accuracy, as well as assessing the competency of the person performing the test. One such approach is the use of a reference sample or control—a test sample in which the chemical composition and physical characteristics simulate the test samples to be analyzed. Hence, a control can be a urine sample which has been kept in the frozen state, or perhaps it comprises pooled urine which has been concentrated through freeze drying, later to be diluted to a predetermined volume.

Exemplary of a commercially available control is Tek-Chek®, marketed by the Ames Division of Miles Laboratories, Inc., which utilizes the effect of a certain pH indicator in the presence of the buffering substance used in commercially available reagent strips having ketone-responsive reagent areas. Using this ketone substitute, Tek-Chek produces a control solution which yields a positive test for ketones with the following Ames Division products: Bili-Labstix®, Labstix®, Keto-Diastix®, Ketostix®, Multistix®, N-Multistix® and Acetest®. Tek-Chek is described in product literature available from the Ames Division as comprising lyophilized urine containing a chemical substitute for ketones. A substitute is used because ketones are difficult to retain in their natural state. Hence, Tek-Chek utilizes a pH indicator to simulate a urine containing pathological amounts of ketone bodies.

Other commercially available ketone control solution products are marketed by Warner-Lambert Pharmaceutical Co. and American Hospital Supply Co., both of which products are liquids and both of which employ acetone as active agent for ketone. Another product is KovaTrol available from I.C.L. Scientific of Fountain Valley, California, which product must be refrigerated until use.

U.S. Patent No. 3,920,400, issued to Scheibe, et al., discloses a uric acid standard solution wherein a lithium salt of uric acid is employed as the control substance, and a complexing agent for polyvalent metals in their higher oxidation states is also present. Typical complexing agents are specified to be malonic acid, salicylic acid, oxalic acid, glutathione, cysteine, 8-oxyquinoline and ethylene-diaminetetraacetic acid. The purpose of the complexing agent additive is to stabilize and prevent the decomposition of uric acid while in solution.

Still another example of a control is that disclosed in U.S. Patent No. 3,920,580, issued to Mast and assigned to the present assignee. There is disclosed a liquid control for glucose determination blood or serum. It comprises water, glucose and an antidiffusing agent comprising a hydrophilic polymer.

Certain salts of cholesterol hemisuccinate are described as being useful for cholesterol controls in U.S. Patent No. 3,859,047. German Offenlegungsschrift 27 21 681 is directed to a device for preparing ketone control solutions utilizing certain metal ion complexes of acetylacetone and its homologs as a substrate (see also US—A—4 193 766). Whereas some ketonesensitive reagents will not respond to such acetylacetonates, other, less specific ones will. It is with the latter ketone reagents that acetylacetonates find their ability as control substrates.

A recent breakthrough in the preparation of urine controls according to an earlier proposal of the applicants (European Patent Application No. 00 29 915 claiming priority of 05.11.1979) is the use of alkali metal enolate salts of $\beta$-ketoalkanoic acid esters for forming control solutions of the corresponding $\beta$-ketoalkanoic acids for use as control solutions with ketone body assays.

To summarize the state of the art prior to the present invention, numerous control solution ingredients are known. Tek-Chek solutions provide a substitute for ketones, a known pH indicator, which reacts with the buffering substances used in various ketone-responsive chemistries normally used in ketone determinations. Other controls are equally known, such as for uric acid, glucose, cholesterol, and many others. Several liquid systems are presently marketed which contain acetone. None of the prior art controls, however, makes known the concepts presently disclosed and claimed. None discloses a ketone control device which utilizes the combination of a $\beta$-ketoalkanoic acid ester and the metal anions disclosed herein.

The present invention departs from the state of the art in dramatic fashion. No longer is it necessary to employ liquid formulations containing acetone or other liquid ketone. The present invention utilizes as an active ingredient a dry, easily storable material which, when dissolved, is directly reactive with the reagents of a ketone-sensitive test. Moreover the invention produces a $\beta$-ketoalkanoic acid *in situ*. The invention eliminates the need for liquid reagents and/or substitutes for ketones such as pH indicators responsive to the buffer of the ketone-sensitive reagent system. Thus, the invention provides a dry device, one which is stable upon storage and easily handled, and which provides ketone control solutions of remarkably accurate concentrations, ergo reproducibility with the ketone-sensitive test procedure.

## Summary of the invention

Briefly stated, the present invention comprises a device for use in the preparation of a control solution for ketone analysis and a method for preparing it. The device comprises a carrier matrix incorporated with a Group II metal or aluminum salt of a $\beta$-ketoalkanoic acid ester. When a specific amount of the salt is contacted with a known volume of water the ester salt is

converted to a solution of the $\beta$-ketoalkanoic acid in known concentration. The ester salt has the structure

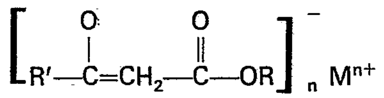

in which R is a $C_1$- to $C_6$-alkyl group, R′ is an aliphatic or cyclic group having 1 to 7 carbon atoms, M is a metal ion from Group II of the Periodic Table of the Elements or aluminum, and n is 2 or 3. The method comprises contacting a predetermined volume of solvent with the device.

Detailed description of the invention

As used herein, the term "carrier matrix" is intended to include any means suitable for transporting a specified amount of the ester and hydrolyzing substance. It can comprise a wide range of materials. The carrier matrix is incorporated with a known amount of the ester salt and, when used, is immersed in a predetermined volume of water for a predetermined time, and removed, leaving the ingredients behind in solution.

The carrier matrix can comprise any substance capable of being incorporated with the ingredients. Thus the matrix can take on many known forms such as those utilized for reagent strips for solution analysis. For example, U.S. Patent No. 3,846,247 teaches the use of felt, porous ceramic strips, and woven or matted glass fibers. As substitutes for paper, U.S. Patent No. 3,522,928 teaches the use of wood sticks, cloth, sponge material, and argillaceous substances. The use of synthetic resin fleeces and glass fiber felts in place of paper is suggested in British Patent No. 1,369,139. Another British Patent No. 1,349,623, suggests the use of a light-permeable meshwork of thin filaments as a cover for an underlying paper matrix. This reference also suggests impregnating the paper with part of a reagent system and impregnating the meshwork with other potentially incompatible reagents. French Patent No. 2,170,397 teaches the use of carrier matrices having greater than 50% polyamide fibers therein. Another approach to carrier matrices is disclosed in U.S. Patent No. 4,046,513 wherein the concept of printing reagents onto a suitable carrier matrix is employed. U.S. Patent No. 4,046,514 discloses the interweaving or knitting of filaments bearing reagents in a reactant system. All such carrier matrix concepts can be employed in the present invention, as can others. Preferably the carrier matrix comprises a bibulous material, such as filter paper, whereby a solution of the ester and hydrolyzing substance is used to impregnate the matrix. It can also comprise a system which physically entraps these ingredients, such as polymeric microcapsules, which then rupture upon contact with the test sample. It can comprise a system wherein the ingredients are homogeneously combined with the carrier matrix in a fluid or semi-fluid state, which later hardens or sets, thereby entrapping the ingredients.

As stated *supra*, the ester salt of the present invention comprises one of a $\beta$-ketoalkanoic acid. In the structure of the ester depicted above R is meant to include any alkyl group having from 1 to 6 carbon atoms, including methyl, ethyl, n-butyl, sec-butyl, tert-butyl, isobutyl, cyclobutyl, and all pentyl and hexyl isomers. R′, on the other hand, can take on much broader significance. It can comprise an aliphatic group or a cyclic group having 1 to 7 carbon atoms. These can be saturated, unsaturated, aromatic, substituted or unsubstituted. Especially suitable for the present invention are salts of such esters as methyl acetoacetate, ethylacetoacetate and ethyl 3-keto-4-phenylbutyrate. Still others are 1,10-di(acetoacetyl)decane, ethyl 2-methylacetoacetate, and ethyl p-fluorobenzoyl acetate. Salts of many additional $\beta$-ketoalkanoic acid esters are within the scope of the present invention, the only limiting requisites being that the ester salt is hydrolyzable in the presence of water to produce the corresponding $\beta$-ketoalkanoic acid.

The amount of $\beta$-ketoalkanoic acid ester salt utilized in the present invention, i.e., incorporated with the carrier matrix, depends upon several parameters. Firstly, the particular system in which ketone body presence might be of analytical interest demands an analytical system responsive to a certain range of ketone concentration. This concentration range will vary from system to system. Pathological urines, for example, necessitate that the ketone-sensitive area of N-Multistix be responsive to ketone concentrations of from about 3 up to about 160 milligrams per deciliter (mg%). Accordingly, for a device for preparing a control solution for N-Multistix reagent strips, an amount of ester salt sufficient to provide a color change indicative of ketone concentrations in that range is required.

A second determining factor is the volume of control solution the device will ultimately be used to prepare. Thus if the device is incorporated with amounts of ingredients which when contacted with 30 milliliters of water will provide the desired N-Multistix reagent strip response, that same device will provide too strong a response with 12 milliliters of water and too weak a response with one liter. Suffice it to say that the amount of ester salt incorporated with the carrier vehicle must be at least sufficient to provide the desired ketone concentration range in a predetermined amount of solvent. In urinanalysis procedures, that concentration range is from about 0.1 to about 160 millimoles per liter.

Thus, the composition can comprise a metal enolate having the structure

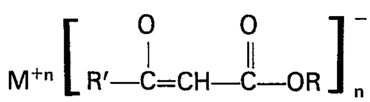

wherein R′ and R are as defined, supra; M is an ion of a Group II metal, including magnesium, calcium, strontium and barium or aluminum of Group III; and the value of n is 2 or 3.

In a preferred method for preparing the test device, filter paper is impregnated with an organic solution or suspension of the metal enolate. Thus, a strip of filter paper is immersed in a solution of the β-ketoalkanoic acid ester salt and dried. With the paper matrix thus incorporated with the active ingredient, it is then attached to a rigid or semi-rigid support such as polystyrene film. A double-faced adhesive tape, such as Double-Stick (3M Company) has been found especially suitable for this purpose. Before mounting, the impregnated paper is cut into narrow strips. These are mounted on polystyrene film along one edge using the double-faced adhesive. The filter paper/polystyrene composite is then slit along lines perpendicular to the axis of the filter paper, thus providing an oblong polystyrene strip having a piece of impregnated filter paper at one end, the other end serving as a handle.

## Examples

The following examples describe experiments which were performed in developing the present invention and/or which illustrate preferred embodiments thereof. While the examples serve to illustrate the invention, they are in no way to be interpreted as limiting its scope.

### Example I
### Calcium methyl acetoacetate

Experiments were conducted to prepare the calcium salt of methyl acetoacetate (calcium methyl β-ketopropionate). Two methods were utilized to prepare this salt; an aqueous method and one utilizing methanol.

#### a. Aqueous method

In the aqueous procedure, 40.0 grams (g) of methyl acetoacetate (Aldrich Chemical Co., Milwaukee, WI) were added dropwise, with stirring to a solution containing 38.0 g of calcium nitrate tetrahydrate and 25 milliliters (ml) of concentrated ammonium hydroxide in 300 ml of water. At pH 9 a white precipitate formed. The precipitate was immediately filtered to avoid hydrolysis of the ester groups. The precipitate was washed with three 25 ml portions of anhydrous ether and dried in an oven at 90°C for 2 hours. The yield after drying was 33.92 g (78.01%) of powder which had a slightly orange tint. The material was soluble in water and methanol (1 mg/ml), was insoluble in carbon tetrachloride and acetone, and was slightly soluble in ethanol. The compound was reprecipitated by dissolving it in dimethylformamide or dimethyl-sulfoxide, followed by the addition of methanol until precipitation occurred. No melting point could be observed. The product gave the following data from proton magnetic radiation (PMR) analysis in Unisol, a nonaqueous solvent sold by Norell Chemical Co. of Landisville, NJ., and comprising a mixture of methylene chloride, dimethyl sulfoxide and chloroform:

1.8 parts per million (ppm), singlet (s)
7.7 ppm, s
3.5 ppm, s
4.6 ppm, s

A 10 milligram (mg) sample of product was suspended in water, shaken and allowed to stand for 30 minutes. The supernatant liquid gave a positive test with Ketostix.

#### b. Methanol procedure

In the methanol procedure, 65.0 g methyl acetoacetate was added slowly, with stirring, to a solution of 80.0 g calcium nitrate tetrahydrate crystals and 40 ml concentrated ammonium hydroxide in 175 ml methanol. A powdery white precipitate formed at pH 10, which was collected by vacuum filtration. The filter cake was washed three times with 25 ml portions of anhydrous ether, and dried in an oven at 90°C for two hours. No melting point could be observed, and the material turned orange and decomposed at 260°C. The solubility in water of the calcium methyl acetoacetate prepared by this procedure was found to exceed that of the above-described aqueous procedure, but the yield was somewhat lower (30.5%, as compared to 78% in the aqueous system).

The compound was suspended in water (1 mg/ml) and tested with Ketostix after 5 minutes. A strong positive result was obtained, indicating rapid hydrolysis to acetoacetic acid.

### Example II
### Barium methyl acetoacetate

The barium salt of methylacetoacetate was prepared using a methanolic system. Barium hydroxide octahydrate (6.3 g) was added to a solution of 11 ml methyl acetoacetate in 100 ml methanol. The mixture was stirred for 5 to 10 minutes whereupon a white curd precipitate began to form. When all of the barium hydroxide had disappeared, the reaction mixture was filtered by vacuum filtration. The filter cake was washed three times with ether and dried in an oven at 90°C for one hour. No melting point was observable, and decomposition to a brown residue was observed at 220°C.

To test the efficacy of the barium salt of methyl acetoacetate in forming a ketone control solution, filter paper devices containing the salt were prepared. A solution containing 5 grams of the barium salt per 100 ml in dimethyl sulfoxide was prepared (5% w/v). Strips of

Whatman filter paper measuring 5 mm (millimeters) by 100 mm were immersed briefly in the solution, removed, and dried for 1 hour at 90°C in an oven. One of the dried strips was immersed and shaken in 10 ml water, allowed to stand for ten minutes and shaken again. The control solution thus prepared gave an immediate positive test with a Ketostix test strip.

Example III
Strontium methyl acetoacetate

Strontium methyl acetoacetate was prepared by adding 22 ml methyl acetoacetate, with stirring to a solution of 6.3 g anhydrous strontium nitrate and 20 ml concentrated ammonium hydroxide in a mixture of 100 ml water and 100 ml methanol. After about 10 minutes stirring, the reaction mixture became cloudy and a precipitate began to form. Upon chilling, a white precipitate accumulated, which was collected by vacuum filtration. The precipitate was washed three times with ether and dried in an oven at 90°C for one hour. No melting point could be observed for the dried precipitate. At 220°C it began to decompose and turn yellow. At 225—230°C it turned orange. The yield was 5.16 g (53%).

To study the utility of the thus-prepared strontium methyl acetoacetate as a substrate for a ketone control solution, 10 mg of the dried precipitate was suspended in 10 ml water, shaken and allowed to stand for five minutes. The liquid was tested with Ketostix and a strong positive test for ketone bodies was obtained.

Example IV
Aluminum methyl acetoacetate

An experiment was conducted to prepare aluminum methyl acetoacetate, and to evaluate its efficacy in forming a ketone body control solution. Methyl acetoacetate (7.08 g) was added dropwise to a solution of 5.00 g sodium aluminate in 50 ml water. A white, flocculent precipitate formed which was collected by vacuum filtration, and dried for 3 days at ambient conditions. The crude material was recrystallized from a benzene/ethanol mixture and redried. No melting point was observed. The yield was 3.8 g (51.1%).

A 10 mg portion of recrystallized material was suspended in 10 ml water, shaken, and allowed to stand 5 to 10 minutes. The liquid gave a positive test for ketone bodies when contacted with a Ketostix test strip.

Example V
Magnesium methyl acetoacetate

An experiment was performed to study the efficacy of magnesium acetoacetate in preparing a control solution for ketone bodies. A 10.44 g portion of methyl acetoacetate was added, with stirring, to a solution of 5.00 g magnesium sulfate and 2 ml concentrated ammonium hydroxide in 150 ml water. Upon addition of the ester, the cloudy reaction mixture cleared, followed by formation of a white precipitate. The solid was separated by vacuum filtration, washed three times with ether, and oven dried at 90°C for one hour. The dried compound melted at 238—240°C with slight yellowing. The yield was 3.79 g (35.5%).

A 10 mg portion of the dried product was suspended in 10 ml water, shaken, and allowed to stand for 15 minutes. The liquid gave a positive test for ketone bodies when tested with Ketostix test strips.

**Claims**

1. A device for use in the preparation of a ketone control solution, characterized in that said device comprises a carrier matrix incorporated with a predetermined quantity of a composition comprising a Group II metal or aluminum salt of a β-ketoalkanoic acid ester having the structure

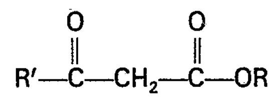

in which R is an alkyl group having 1 to 6 carbon atoms and R′ is an aliphatic or cyclic group having 1 to 7 carbon atoms.

2. A device according to claim 1, characterized in that R′ is methyl.

3. A device according to claim 1, characterized in that ester is methylacetoacetate.

4. A device according to any of claims 1—3, characterized in that the salt is the calcium salt.

5. A device according to any of claims 1—3, characterized in that the salt is the barium salt.

6. A device according to any of claims 1—5, characterized in that the carrier matrix is paper.

7. A device according to claim 1, characterized in that it comprises a carrier matrix incorporated with a predetermined amount of a calcium or barium salt of a ($C_1$- to $C_6$-alkyl) acetoacetate; and a support member comprising an elongated plastic strip having said carrier matrix affixed thereto.

8. A device according to claim 7, characterized in that the salt is the calcium salt of methylacetoacetate.

9. A method for preparing a ketone control solution, characterized in that it comprises contacting a predetermined volume of solvent with a predetermined quantity of a composition comprising a Group II metal or aluminum salt of a β-ketoalkanoic acid ester, said ester having the structure

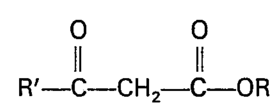

in which R is an alkyl group having 1 to 6 carbon atoms and R′ is an aliphatic or cyclic group having 1 to 7 carbon atoms.

## Patentansprüche

1. Vorrichtung zur Verwendung bei der Herstellung einer Keton-Kontrollösung, dadurch gekennzeichnet, dass die genannte Vorrichtung eine Trägermatrix umfasst, welche mit einer gegebenen Menge eines Mittels inkorporiert ist, welches ein Gruppe-II-Metall- oder Aluminiumsalz eines $\beta$-Ketoalkansäure-esters der folgenden Strukture umfasst:

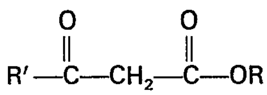

worin R eine Alkylgruppe mit 1 bis 6 Kohlenstoffatomen und R′ eine aliphatische oder cyclische Gruppe mit 1 bis 7 Kohlenstoffatomen darstellen.

2. Vorrichtung gemäss Anspruch 1, dadurch gekennzeichnet, dass R′ Methyl bedeutet.

3. Vorrichtung gemäss Anspruch 1, dadurch gekennzeichnet, dass der Ester Methylacetoacetat darstellt.

4. Vorrichtung gemäss einem oder mehreren der Ansprüche 1 bis 3, dadurch, gekennzeichnet, dass das Salz das Calciumsalz darstellt.

5. Vorrichtung gemäss einem oder mehreren der Ansprüche 1 bis 3, dadurch gekennzeichnet, dass das Salz das Bariumsalz darstellt.

6. Vorrichtung gemäss einem oder mehreren der Ansprüche 1 bis 5, dadurch gekennzeichnet, dass die Trägermatrix Papier darstellt.

7. Vorrichtung gemäss Anspruch 1, dadurch gekennzeichnet, dass sie eine Trägermatrix, welche mit einer gegebenen Menge eines Calcium- oder Bariumsalzes eines ($C_1$- bis $C_6$-Alkyl)- acetoacetats unf einen Träger aus einem länglichen Plastikstreifen, auf welchem die Trägermatrix angebracht ist, umfasst.

8. Vorrichtung gemäss Anspruch 7, dadurch gekennzeichnet, dass das Salz das Calciumsalz von Methylacetoacetat darstellt.

9. Verfahren zur Herstellung einer Keton-Kontrolllösung, dadurch gekennzeichnet, dass ein gegebenes Lösungsmittelvolumen mit einer gegebenen Menge eines Mittels, welches ein Gruppe-II-Metall- oder Aluminiumsalz eines $\beta$-Ketoalkansäureesters umfasst, wobei der Ester die folgende Struktur aufweist:

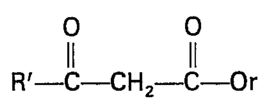

worin R eine Alkylgruppe mit 1 bis 6 Kohlenstoffatomen und R′ eine aliphatische oder cyclische Gruppe mit 1 bis 7 Kohlenstoffatomen darstellen, miteinander in Kontakt gebracht werden.

## Revendications

1. Un dispositif pour l'utilisation dans la préparation d'une solution de contrôle d'une cétone, caractérisé en ce que ledit dispositif comprend une matrice de support dans laquelle est incorporée une quantité prédéterminée d'une composition comprenant un sel de métal de groupe II ou d'aluminium d'un ester $\beta$-cétoalcanoïque de structure

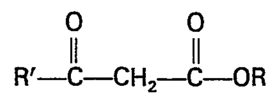

dans laquelle R est un groupe alkyl ayant 1 à 6 atomes de carbone et R′ est un groupe aliphatique ou cyclique ayant 1 à 7 atomes de carbone.

2. Un dispositif selon la revendication 1, caractérisé en ce que R′ est un groupe méthyle.

3. Un dispositif selon la revendication 1, caractérisé en ce que l'ester est l'acétoacétate de méthyle.

4. Un dispositif selon l'une quelconque des revendications 1—3, caractérisé en ce que le sel est le sel de calcium.

5. Un dispositif selon l'une quelconque des revendications 1—3, caractérisé en ce que le sel est le sel de baryum.

6. Un dispositif selon l'une quelconque des revendications 1—5, caractérisé en ce que la matrice de support est le papier.

7. Un dispositif selon la revendication 1, caractérisé en ce qu'il comprend un matrice de support dans laquelle est incorporée une quantité prédéterminée d'un sel de calcium ou de baryum d'un acétoacétate d'alkyl en $C_1$—$C_6$; et un élément de support comprenant une bande de plastique allongée sur laquelle est fixée ladite matrice de support.

8. Un dispositif selon la revendication 7, caractérisé en ce que le sel est le sel de calcium de l'acétoacétate de méthyle.

9. Un procédé pour préparer une solution de contrôle de cétone, caractérisé en ce qu'il consiste à mettre en contact un volume prédéterminé d'un solvant avec une quantité prédéterminée d'une composition comprenant un sel de métal du groupe II ou d'aluminium d'un ester $\beta$-cétoalcanoïque de structure

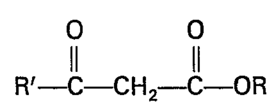

dans laquelle R est un groupe alkyl ayant 1 à 6 atomes de carbone et R′ est un groupe aliphatique ou cyclique ayant 1 à 7 atomes de carbone.